# EUROPEAN PATENT SPECIFICATION

(11) **EP 2 488 230 B1**
(45) Date of publication and mention of the grant of the patent: **28.08.2013**
(21) Application number: 10766212.4
(22) Date of filing: 13.10.2010
(51) Int. Cl.: A61M 5/145, A61M 5/168, A61M 5/142, H02H 7/085

(54) **OCCLUSION RECOGNITION IN AN ADMINISTERING APPARATUS**
OKKLUSIONSERKENNUNG IN EINEM VERABREICHUNGSGERÄT
DÉTECTION D'OCCLUSION DANS UN APPAREIL D'ADMINISTRATION

(30) Priority: 16.10.2009 CH 15902009
(43) Date of publication of application: 22.08.2012
(73) Proprietor: Tecpharma Licensing AG, 3401 Burgdorf (CH)
(72) Inventor: BURI, Thomas, CH-3400 Burgdorf (CH); RUFER, Michael, CH-4574 Lüsslingen (CH)
(86) International application number: PCT/CH2010/000253
(87) International publication number: WO 2011/044706

(56) References cited:
- EP-A2- 0 341 364
- GB-A- 2 447 171
- US-A1- 2003 171 712
- US-A1- 2007 149 926
- US-A1- 2009 043 240
- US-B1- 6 362 591

## Description

### TECHNICAL FIELD

The present invention relates to a device and a method for controlling a medical administering apparatus, which allows for an improved recognition of occlusions. The administering apparatus can be, e.g., an infusion apparatus that is used to administer a fluid medicament to a patient over a relatively long time.

### PRIOR ART

With various diseases, it may be necessary to administer to a patient a medicament present in fluid form, e.g. an insulin preparation or a blood-thinning medicament such as Heparin, over a relatively long time. Compact, portable, body-external administering apparatuses, which are permanently carried by the patient in the vicinity of the body, are known for this purpose. Such an administering apparatus is specified, e.g., in WO 2008/106806.

In such administering apparatuses, the medicament is often present in a cartridge-like reservoir, in which a piston is advanced in order to release the medicament from the reservoir. The piston is advanced with the aid of an electrical drive motor, the rotational movement of which is converted into an axial advance movement of the piston via a transmission and further drive components. The administration occurs intermittently, with relatively short discharging events, during which the motor is activated and hence administration occurs, being separated by relatively long pauses. A complete administration cycle comprises a discharging event, which typically only takes a few seconds, and a subsequent pause, which typically lasts a few minutes.

For a reliable supply of the medicament to the patient it is essential that malfunctions, which can lead to an undersupply or oversupply of the medicament, are effectively and reliably recognized and lead to the triggering of an alarm and/or the apparatus being switched off. More particularly, such a malfunction can result from an occlusion occurring in a liquid-carrying line, e.g. as a result of the infusion set being clogged or bent over. If the drive motor is activated in the presence of an occlusion, this generally leads to a pressure increase in the reservoir. When the occlusion is once again removed, the pressure is again reduced in the reservoir by the medicament flowing out of the reservoir. Thus, an occlusion can at first lead to an undersupply of the medicament, while after the removal of an occlusion that has persisted over an extended period of time an undesired large amount of the medicament might possibly flow out. If the medicament is, e.g., insulin, this can, in an extreme case, lead to life-threatening hypoglycaemia. It is therefore of the utmost importance that occlusions are recognized early such that the patient is warned in good time and can undertake suitable measures.

In order to recognize occlusions, the prior art has proposed various measures, including methods based on measuring parameters of the drive motor, more particularly the electrical current flowing through the drive motor. This is based on the fact that in many types of electromotors (in particular, commercially available DC motors), the emitted torque is substantially proportional to the electrical current flowing through the motor, or at least increases continuously and monotonically with the current. If the fluid pressure increases in the reservoir, so does the force that needs to be applied for a further advance of the piston. Thus, a higher torque is required for a further advance, which in turn is expressed as an increase in the current flowing through the motor. This affords the possibility of recognizing an occlusion.

A method for recognizing occlusions based on this principle is specified e.g. in US 2007/0191770. The motor current is monitored. If the motor current exceeds a normal base-line value by a certain threshold, an occlusion alarm is triggered.

However, the method specified therein presupposes that the motor has reached a state with an almost constant rotational speed (a stationary state). During the acceleration or deceleration of the motor, the inertia in the system causes an additional torque, which falsifies the measurement. This can cause false alarms, in particular during short discharging events, in which the motor is accelerated over a significant part of the discharging event and decelerated again.

US2003/0171712 discloses an injector system according to the preamble of claim 1, and in which the electrical current is limited in order to limit pressure within a syringe having a movable plunger. The plunger is driven by a drive arrangement having an electrical motor to produce a torque which is translated into a linear force on the plunger by a drive screw. The relation between electrical current and torque produced by the motor of the injector is modeled using differential equations depending on velocity and acceleration. However, the described methods exhibits a need for a complicated calibration sequence which itself has to be periodically repeated by performing movements of the syringe plunger in an empty syringe. The procedure has to be performed for every velocity and acceleration.

### DESCRIPTION OF THE INVENTION

It is therefore an object of the present invention to provide a device for controlling a medical administering apparatus, which device allows simple and reliable recognition of occlusions, in particular, even for short discharging events in which the motor is accelerated or decelerated again over a significant part of the discharging event. This object is achieved by a device with the features of Claim 1.

The present invention additionally provides an administering apparatus with the features of Claim 14, equipped with such a device.

Preferred embodiments are specified in the dependent claims.

Thus, according to a first aspect of the invention, a device for controlling a medical administering apparatus is specified, which has the following features:
a motor control arrangement designed to activate an electrical motor of the medical administering apparatus during defined discharging events, the electrical motor forming a (variable) electrical load for the motor control arrangement;
a load sensor for determining load signals that constitute a measure for the electrical load formed by the motor;
a monitoring arrangement designed to compare a variable derived from the load signals and at least one predefined condition, and to emit an occlusion signal if the condition is satisfied.

This device is distinguished by virtue of the fact that the motor control arrangement is designed to actuate the motor as per a predefined speed profile, i.e. to actuate the motor such that the motor velocity generated by the motor (more precisely: the angular velocity of the motor shaft) substantially follows the speed profile. The monitoring arrangement has a correction module for correcting, by means of associated region-dependent correction values, the load signals as a function of the region of the speed profile in which the motor control arrangement is actually in. This can occur before or after further processing of the load signals.

This affords the possibility of taking into account the influence of the inertia on the load signals during the acceleration or deceleration of the motor. When the motor accelerates, it requires more power than if it is in a stationary state with a constant rotational speed. This leads to increased load signals. By contrast, the load signals are smaller during braking or slowing-down of the motor (i.e. during negative acceleration) than during a stationary state. The amount by which the load signals should be corrected in the case of a given acceleration or deceleration of the motor (expressed more generally, in a given region of the speed profile) can be established experimentally by measuring the load signals for the different regions of the speed profile when it is known that there is no occlusion present. These values can be established for each administering apparatus, e.g., only once during production, or when the apparatus is put into operation for the first time, or, e.g., automatically after each reservoir change, and said values can be stored as correction values in a memory of the device. The correction values can also be the same for an entire apparatus batch, be established once for the entire batch and be stored in the apparatuses of an entire batch.

In particular, the load signals can constitute a measure for the electrical current flowing through the motor. Alternatively, or in addition thereto, the load signals can however also represent the electrical power taken up by the motor. Other variables are also feasible as load signals, e.g. the electrical voltage across the motor, which drops at the motor at a predefined rotational speed, etc. Expressed in general terms, any electrical variable of the motor that allows conclusions to be drawn about the torque emitted by the motor is suitable as a load signal.

In particular, the device can have a memory for storing the speed profile and in each case at least one associated correction value for each region of the speed profile. The motor control arrangement is designed to read out the speed profile from the memory and actuate the motor as per the speed profile. The correction module is designed to read out the correction values from the memory and to correct the load signals accordingly.

In the simplest case, the speed profile has at least the following regions:
an acceleration region in which the motor speed substantially increases, preferably linearly with time (constant acceleration); and
a deceleration region in which the motor speed substantially decreases, preferably likewise linearly with time (constant deceleration).

In between, there can be a stationary region, in which the motor speed is substantially constant; however, this stationary region can be dispensed with, e.g. in the case of very short discharging events.

A first (constant) correction value for the load signals is then associated with the acceleration region and a second (constant) correction value for the load signals is associated with the deceleration region. Each of these correction values is a constant, which has been established, e.g., only once already during production, when the administering apparatus is first put into operation, or after each reservoir change, and stored in the memory. By correcting the load signals of the acceleration region by a first correction value and the load signals of the deceleration region by a second correction value, it becomes possible to detect an increase in load, possibly caused by an occlusion, reliably, even if the administering events are very short such that the motor does not even reach a stationary state during the administering event.

It goes without saying that the speed profile can however contain additional regions. More particularly, it can also be quasi-continuous, i.e. approximated by a multiplicity of successive short regions, e.g. by each of these regions being defined by a time and an associated speed value, and interpolation taking place between these speed values (sampling). It is not mandatory for the profile to contain a stationary region. Even for such a general profile, the correction of the load signals allows a more reliable detection of an increased load on the motor, which could indicate an occlusion.

The device preferably also comprises a position sensor for establishing an actual position of the motor or a drive component connected thereto, e.g. a transmission element driven by the motor, and/or a speed sensor for establishing the motor speed. The sensor can be e.g. an encoder, e.g. a known optical, magnetic or other encoder, which affords the possibility of measuring both the actual position and the motor speed. Alternatively, or in addition thereto, it is also possible to use a voltage sensor as a sensor for measuring the back EMF on the motor, which likewise allows conclusions to be drawn in respect of the motor position and/or motor speed.

This firstly affords the possibility of actively regulating the motor speed by a feedback control loop. Thus, in this case, the control arrangement has a speed controller, which receives the established motor speed as a control variable and intended speed values as per the speed profile as a reference variable and outputs a manipulated variable for the motor for regulating the motor speed as per the speed profile.

Secondly, this affords the possibility of changing the motor speed (possibly regulated by the speed controller) as a function of the motor position (rather than, e.g., as a function of time). In this case, it can be particularly advantageous if the speed profile is at least in part defined by virtue of the fact that intended speed values are predefined as a function of a position deviation of the actual position from an intended position. In particular, the intended position can be the target position that the motor should reach at the end of a discharging event. This allows a targeted reduction in the motor speed at the end of the discharging event in order to avoid an overrun of the motor, after switching off the motor, without actively braking the motor (more particularly without a short-circuit brake). In particular, the speed profile can be defined by virtue of the fact that the speed profile comprises a list containing a plurality of position deviations between the actual position and an intended position, and associated intended speed values.

In order to reduce noise and improve the reliability of occlusion recognition, a number of measures can be taken independently or cumulatively. Firstly, it is possible for a low-pass filter to be provided for the load signals in order to filter out fast changes in the load signals. Secondly, the monitoring arrangement preferably comprises an averaging module for forming averages from the load signals. The term "average" should be understood in broad terms in this case and should also comprise the (weighted or unweighted) sum of a plurality of load signals at different times. The averaging can be brought about e.g. by virtue of the fact that the load signals are registered by sampling, e.g. at predefined times or positions of the motor, more particularly after each encoder step, and the sampled load signals are summed over a certain region of samples. Up to a constant factor (the number of samples), this sum corresponds to the arithmetic mean of the samples. In the case of weighted averaging, the load signal samples are additionally multiplied by different weighting factors before the summation.

The averaging module can more particularly be designed to generate the averages in a two-stage method. In a first step, a first average for successive discrete movement regions of the motor (e.g. for a predefined number of revolutions of the motor) is respectively formed in a first submodule. In a second step, second averages are formed in a second submodule by means of a running average from the first averages.

The load sensor is preferably a current sensor for establishing load signals representing a current flowing through the motor. The current sensor can more particularly be implemented as a resistor connected in series with the motor, with an amplifier for a voltage drop across the resistor. Alternatively, or in addition thereto, the current sensor can comprise a Hall sensor for measuring a magnetic field generated by the current. Alternatively, or in addition thereto, it is however also feasible e.g. to measure other electrical parameters of the motor, e.g. the voltage drop across the motor or the electrical power taken in by the motor.

The device can additionally have an alarm arrangement for emitting one or more of the following signals as a function of the occlusion signals:
an optical alarm signal;
an acoustic alarm signal; or
a tactile alarm signal.

In order to increase operational reliability, the device can have a redundant design and, in particular, have a second independent monitoring arrangement for this purpose designed to compare a variable derived from the load signals with at least one predefined condition and to emit an occlusion signal if the condition is satisfied. This second monitoring arrangement may substantially have the same design as the first monitoring arrangement and may likewise for this purpose have a correction module for reading out correction values from the same or from a second independent memory and for correcting, by the associated correction value, the load signals as a function of the region of the speed profile in which the motor control arrangement is situated.

The monitoring arrangements, the control arrangement and the associated modules may be implemented as analogue or digital hardware or in software/firmware. More particularly, an analogue-digital converter (ADC) may be available for the load signals for this purpose, and the motor may be actuated in a digital fashion at the output of the motor control arrangement, e.g. via a well-known pulse-width modulator.

According to a second aspect, the present invention relates to a medical administering apparatus, more particularly an infusion apparatus, for administering a fluid medicament from a reservoir, equipped with a control device of the aforementioned type. Moreover, such an administering apparatus comprises an electrical motor interacting with the control device for generating a drive movement, and one or more drive components, coupled to the motor, for transmitting the drive movement onto the reservoir in order to eject the medicament from the reservoir. The motor is preferably a DC motor.

According to a third aspect, the present invention provides a method for controlling a medical administering apparatus. The administering apparatus comprises a reservoir for a fluid medicament, an electrical motor for generating a drive movement, the motor forming an electrical load, and one or more drive components, coupled to the motor, for transmitting the drive movement onto the reservoir. The method has the following steps:
activating the electrical motor during defined discharging events separated by pauses such that the drive movement generated by the motor has a motor speed substantially following a predefined speed profile with a plurality of regions;
establishing load signals during the discharging events, with the load signals constituting a measure for the electrical load formed by the motor;
correcting the load signals by correction values which are dependent on the region of the speed profile in which the motor is situated;
comparing a variable derived from the load signals with at least one predefined condition; and
emitting an occlusion signal if the condition is satisfied.

The same considerations in respect of the method apply analogously like the aforementioned considerations in respect of the device for controlling an administering apparatus. More particularly, the speed profile can, as described above, have at least one acceleration region, one deceleration region and optionally one stationary region, with appropriate correction values being associated with the acceleration region and the deceleration region. However, the speed profile can also be more complicated, with further regions.

As already described above, the speed profile can at least in part be prescribed by intended speed values as a function of a position deviation of the actual position from an intended position. Then the method comprises the following steps:
establishing the actual position of the motor, and
determining intended speed values as per the speed profile as a function of a position deviation of the actual position from an intended position.

The motor speed can, as already described above, be regulated actively with feedback. For this, the following steps are performed:
establishing the motor speed; and
regulating the motor speed as per the speed profile by means of a feedback control loop, which receives the established motor speed as a control variable and intended speed values as per the speed profile as a reference variable and outputs a manipulated variable for the motor.

The load signals preferably represent the electrical current flowing through the motor.

As described above, averages or sums over a certain number of samples can be formed from the load signals, more particularly in the aforementioned two-stage manner with the following steps:
forming a first average over a discrete movement region of the motor in each case; and
forming second averages from the first averages by means of a running average.

More particularly, an occlusion signal can be emitted if at least one of the following conditions is satisfied:
a variable derived from the load signals exceeds a predefined absolute value; and
the difference between two variables derived from the load signals at different times exceeds a predefined difference value.

### BRIEF DESCRIPTION OF THE DRAWINGS

In the following, preferred embodiments of the invention are described with reference to the drawings, which merely serve for explanation and should not be construed as being limiting the invention. In the drawings:
- Fig. 1: shows a reusable base unit of a medical administering apparatus in a very schematic sectional illustration;
- Fig. 2: shows a disposable cartridge that can be connected to the base unit of Figure 1;
- Fig. 3: shows a schematic functional diagram of an administering apparatus according to the invention;
- Fig. 4: shows a schematic illustration of a speed profile with three regions;
- Fig. 5: shows a diagram illustrating the definition of an end region of a speed profile as a function of a position deviation;
- Fig. 6: shows a functional diagram of a monitoring arrangement; and
- Fig. 7: shows a diagram illustrating measured speed and current values.

### DESCRIPTION OF PREFERRED EMBODIMENTS

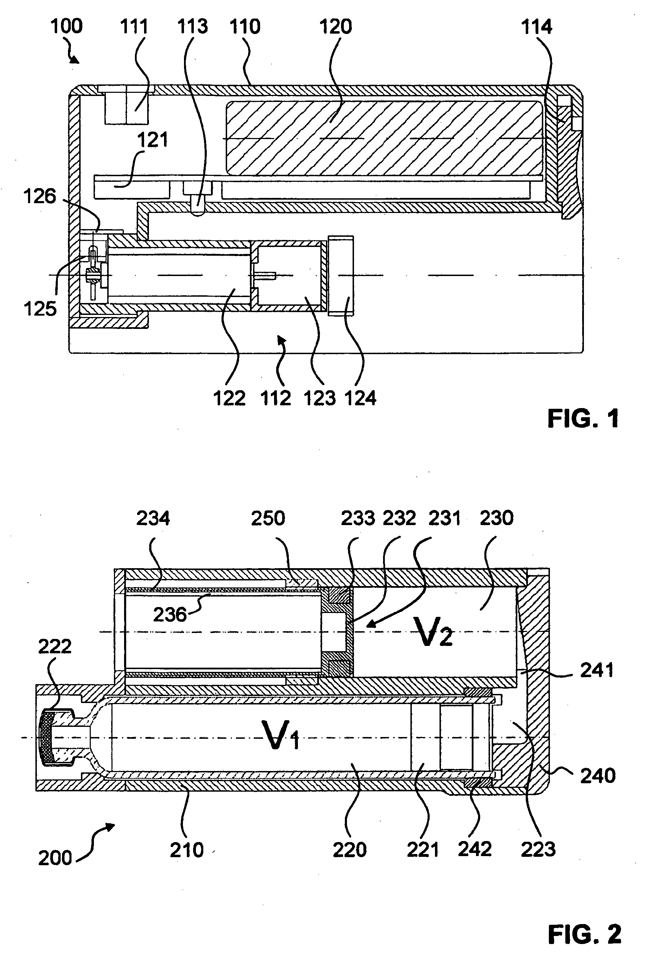

Figure 1 illustrates in an exemplary and schematic fashion a base unit ("reusable module") of a modular administering apparatus ("semi-disposable apparatus") for administering a liquid medicament. A base unit having, in principle, a very similar design has been described in the aforementioned international application WO 2008/106806, to which reference is made in respect of further details of the design of the base unit and the force transmission between the base unit and an associated cartridge.

The base unit 100 has a housing 110, on or within which provision is made, *inter alia,* for an energy source in the form of a battery 120, an electronic control device 121 (indicated only schematically here), an electrical drive motor 122 in the form of a commercially available DC motor and further drive components including a transmission 123 and a driver 124. An encoder 125 is used to register the actual position of the drive motor in the form of the angle of rotation covered by the shaft of the drive motor and, derived from this, the motor speed. Operating elements 111 are arranged on the external side of the housing 110 and are only indicated very schematically here. Such operating elements can comprise, e.g., a display known per se, e.g. an LCD display, and one or more operating buttons. The control arrangement 121 can be operated in respect of the individual requirements of the patient by means of these operating elements.

The motor 122, the transmission 123 and the driver 124 are parts of a finger-like structure 112 with a substantially circular-cylindrical basic shape, with the driver being arranged in the region of the free end of this finger-like structure. The motor 122 drives the driver 124 to carry out a drive rotational movement via the transmission 123. The driver 124 substantially consists of a wheel, on the circumferential surface of which a plurality of driving ribs are arranged extending in the axial direction. The reaction torque taken up in the process by the stator of the motor is transmitted to the housing via a suspension 126 only indicated schematically in Figure 1.

Figure 2 illustrates a replaceable cartridge ("disposable module"), which can be connected to the base unit in Figure 1 and a needle adaptor (not illustrated) to form a complete administering device.

Directional references as indicated in the following are used to specify directions within the cartridge. The distal direction is respectively understood to be that direction in which a relevant moveable element moves during the administration of the product. In the cartridge, there is a deflection of an advance movement about 180° in the interior of the administering apparatus. The distal direction is therefore only defined locally and can correspond to different absolute spatial directions in different parts of the administering apparatus. The proximal direction is in each case defined as the opposite direction to the distal direction. A lateral direction is a direction perpendicular thereto.

The cartridge 200 comprises a housing 210, which, in the region thereof illustrated at the bottom in Figure 2, houses a product container 220 in the form of a cartridge with a cylindrical sidewall region and a product piston 221 that can be displaced therein. At its distal end, the product container 220 is closed off by an end cap 222 with a septum and thus forms a product reservoir with a volume V₁. On its proximal end, the product container is held in a sealing ring 242. A hydraulics reservoir 230 is formed antiparallel to the container 220. In the proximal direction, the hydraulics reservoir 230 is delimited by a hydraulics piston 231, which can move in the axial direction and is guided in a sealing fashion in a sidewall region of the housing. The hydraulics reservoir 230 is connected to a displacement reservoir 223 via a fluid channel 241, which is delimited by a closure element 240. Said displacement reservoir is delimited in the distal direction by the product piston 221. A suitable hydraulics fluid, for example coloured, deionised water, a suitable oil or another incompressible fluid, is filled into the hydraulics reservoir 230, into the displacement reservoir 223 and into the fluid channel 241. Overall the hydraulics fluid takes up a volume V₂.

The hydraulics piston 231 comprises a rigid support 232, on which an annular seal 233 is arranged, which seals the hydraulics piston 231 against the sidewall of the housing. The support 232 merges into a transmission sleeve 234. The transmission sleeve 234 firstly has a male thread, which engages with the female thread of a guide nut 250 arranged fixedly in the housing. Secondly, on its inner lateral face, the transmission sleeve 234 has a plurality of longitudinal grooves 236, which run parallel to the longitudinal direction of the transmission sleeve and are designed to complement the corresponding longitudinal ribs on the driver 124 of the base unit 100. While the transmission sleeve 234 is in this case designed integrally with the hydraulics piston 231, these parts can also be designed to be separate and, more particularly, can be rotatable with respect to one another.

In order to put the administering device into operation and subsequently administer the medicament, a needle adaptor is first of all put onto the cartridge 200, with a catheter of an infusion set connecting to the former. The infusion set ends in a cannula to puncture the skin of a patient. The needle adaptor comprises a hollow needle, which pierces the septum of the end cap 222 of the product container 220 and thus connects the interior of the product container to the catheter. Thereupon the cartridge 200 is connected to the base unit 100. In the process, the finger-like structure 112 is inserted into the interior of the transmission sleeve 234, with the longitudinal ribs on the outer side of the driver 124 engaging with the longitudinal grooves 236 in the inner lateral surface of the transmission sleeve 234. Subsequently the cartridge 200 and the base unit 100 are locked together by means of a latch 114 or another suitable locking device. By means of a switch 113, the control arrangement determines whether the cartridge 200 is correctly connected to the base unit 100. If this is not the case, the device cannot be put into operation.

During normal operation, a certain amount of product is now dispensed from the product container at predefined intervals. For this, the control device 121 actuates the motor 122, and so the motor 122 sets the driver 124 into a rotational movement via the transmission 123. This rotational movement is transmitted onto the longitudinal grooves in the transmission sleeve 234 as a result of the driver 124 engaging therewith. Since the transmission sleeve 234 engages with the guide nut 250 via a threaded connection, the rotational movement at the same time brings about an advance movement of the transmission sleeve 234 (that is to say overall a helical movement in the distal direction) and hence an advance of the entire hydraulics piston 231 in the distal direction. This reduces the volume of the hydraulics reservoir 230, and so the hydraulics fluid is pressed through the fluid channel 241 and into the displacement reservoir 223 and here results in an advance of the product piston 221 in the distal direction.

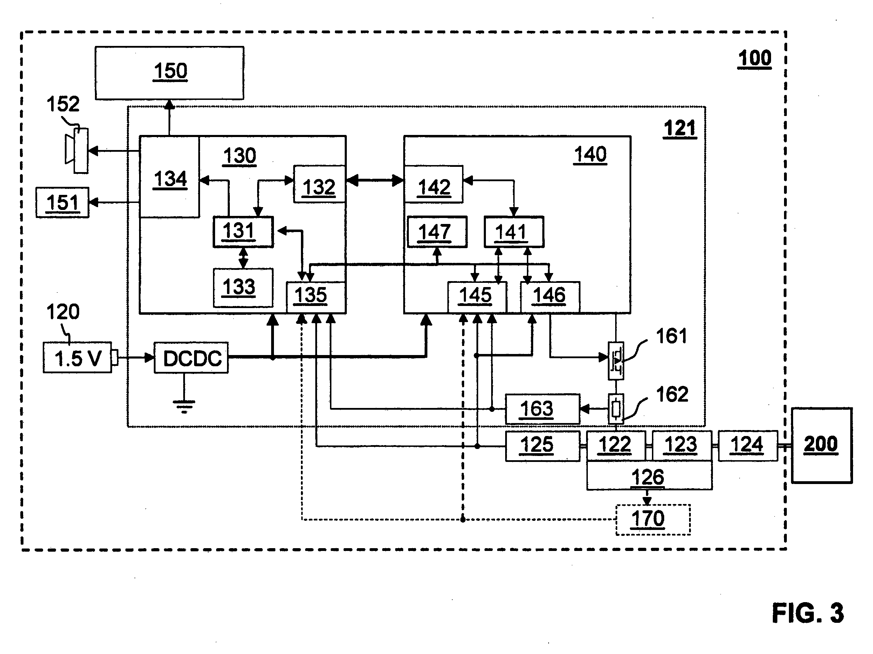

In the following, the control of such an administering apparatus shall be explained in more detail with reference to Figure 3. Parts having the same function have been denoted with the same reference signs as in Figures 1 and 2. However, the type of control explained below is not limited to an administering apparatus as per Figures 1 and 2, but can also be used in any other administering apparatus, more particularly in infusion apparatuses, in which a fluid is ejected from a reservoir with the aid of an electromotor and administered to a patient.

The control device 121 comprises a digital main processor 130 and a digital supervisor processor 140, which are both supplied with voltage from the battery 120 via a DC/DC converter. The supervisor processor 140 comprising a first monitoring arrangement 145 and a motor control arrangement 146, that both interact with a first state machine 141. The state machine 141 communicates with the main processor 130 via a first interface 142. Said main processor comprises a second monitoring arrangement 135, which in turn interacts with a second state machine 131. The second state machine communicates with the first interface 142 via a second interface 132, and hence with the supervisor processor 140. Both monitoring arrangements 135, 145 communicate with a memory 147. The second state machine 131 moreover writes data to a further memory 133, which is used as an event logbook, and interacts with an alarm module 134, which can emit a tactile alarm signal via a vibrator 151, an acoustic alarm signal via a buzzer 152 and a visual (optical) alarm signal via a display 150.

The motor control arrangement 146 actuates a motor driver unit 161, which can have e.g. a MOSFET circuit, which is actuated digitally by means of pulse-width modulation. The motor 122 and the motor driver unit 161 are connected in series to a shunt resistor 162, across which a voltage drops, which, according to Ohm's Law, is directly proportional to the current flowing through the motor 122. This voltage is detected, amplified and low-pass filtered by a voltage detector 163 and digitized by an analogue-digital converter. The shunt resistor 162 and the voltage detector 163 form a load sensor. The monitoring arrangements 135, 145 receive digital load signals from this load sensor, which signals represent the motor current.

The motor 122 acts on the cartridge 200 via the transmission 123 and the driver 124 in order to eject the medicament from the product container 220 (not illustrated in Figure 3). In the process, a reaction torque (expressed generally, a generalized reaction force), which can be registered by an optional force sensor 170, e.g. a strain gauge, acts on the suspension 126. The signals from the force sensor 170 are optionally likewise transmitted to the monitoring arrangements 135, 145, which is indicated in Figure 3 by dashed lines.

The encoder 125 supplies position signals to the monitoring arrangements 135, 145, from which the actual position of the motor and the motor speed can be determined.

During operation, the motor control arrangement 146 intermittently activates the motor 122 at regular intervals for a respective discharging event. There are pauses of a plurality of minutes, e.g. 5-30 minutes, between successive discharging events, while each discharging event in general only takes a few seconds or a few tens of seconds. During each discharging event, a predetermined amount of the medicament is released. For this purpose, the motor is activated until it has carried out an intended number of revolutions, predetermined in advance, in order correspondingly to reach an end position predetermined in advance. The actual number of revolutions carried out during the discharging event is registered by the encoder 125 and compared to the intended value. Possible deviations from the intended value are compensated for in the subsequent discharging event (integral control of the released amount over successive discharging events). If the discharged amount were to drop below a minimum amount during a discharging event, the relevant discharging event is omitted, and the amount that was not discharged is likewise compensated for in the next discharging event

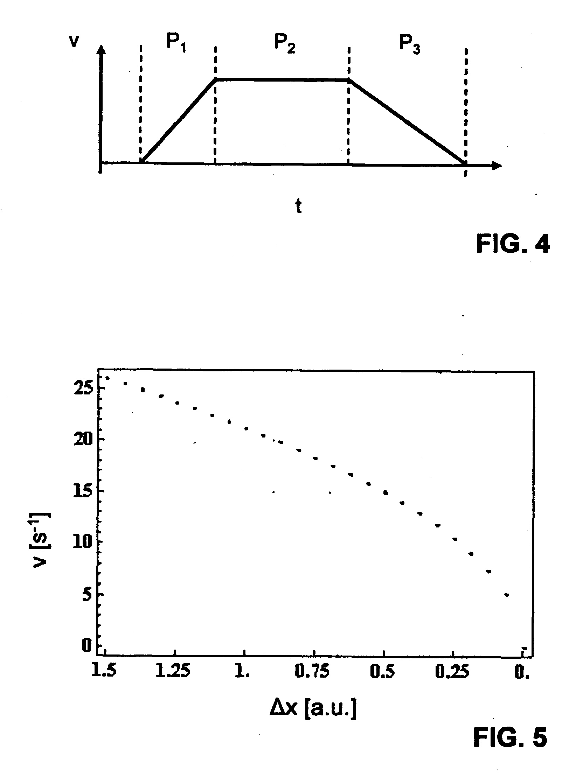

The motor is controlled in each discharging event as follows. A speed profile is stored in the memory 147. The motor control arrangement 146 reads out the speed profile from the memory 147. A simple profile of this type is illustrated schematically in Figure 4. It comprises three phases or regions P₁, P₂, and P₃. In the region P₁, the motor speed v increases linearly with time (region of constant acceleration; start-up ramp). In the region P₂, the motor speed is constant (stationary region). Finally, in the region P₃, the motor speed reduces to zero again linearly with time (region of constant braking or slowing down, i.e. constant negative acceleration; slow-down ramp).

The motor control arrangement 146 actuates the motor as per this profile such that the motor speed substantially follows this profile. For this purpose, the motor control arrangement 146 actively regulates the motor speed by establishing the actual speed from the encoder 125 signals or a back EMF induced from the motor, and matches it to the intended speed predefined by the speed profile with the aid of a controller.

In particular during the slow-down ramp (region P₃), the control of the motor speed is carried out dependent on position,. This is illustrated in Figure 5, which shows a typical slow-down ramp in the form of intended speed values v as a function of a deviation Δx from the desired end position. Here, the link between intended speed v and position deviation Δx is nonlinear and substantially follows a square-root function in order to achieve substantially constant deceleration (constant derivative of speed over time). For this purpose, a table is stored in the memory 147, which firstly contains a plurality of position deviations and secondly associated intended speed values. When the end position is approached, this table is queried by the motor control arrangement as part of the speed profile and is executed in accordance with the actual motor position. The motor speed is almost zero at the end of the discharging event. Further active braking of the motor, e.g. by short-circuiting it, can therefore be dispensed with.

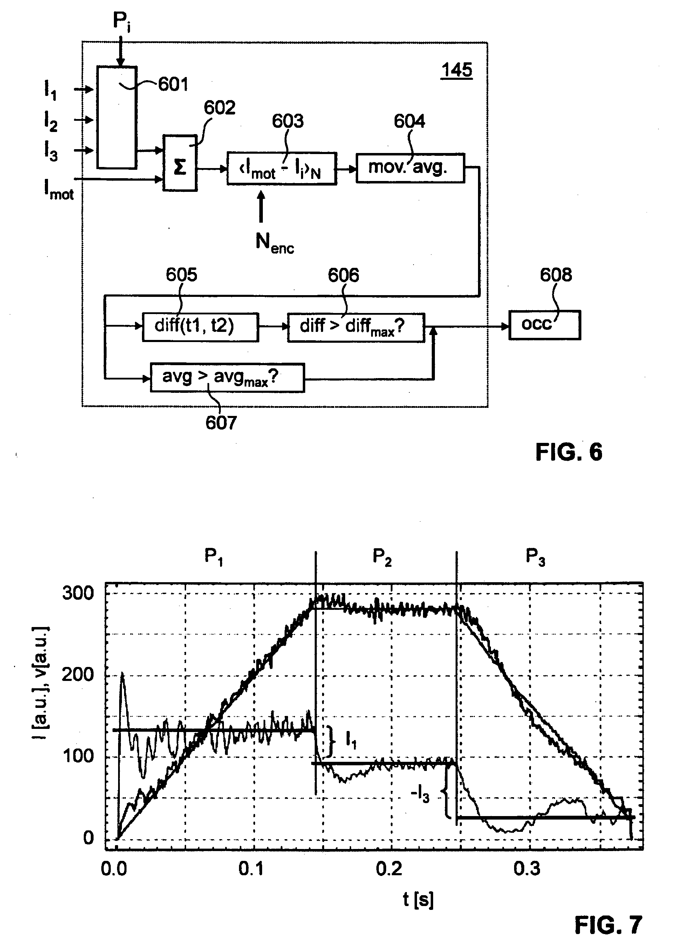

Figure 6 illustrates the functioning of the first monitoring arrangement 145. The second monitoring arrangement has the same basic design.

With the aid of signals Pᵢ, received from the motor control arrangement 146, a selection module 601 determines in which region (P₁, P₂, P₃) of the speed profile the motor 122 is situated. A current-correction value is stored in the memory 147 for each of the regions, which value was established during production before delivery of the administering apparatus and stored in the apparatus. In accordance with the region, the selection module reads out the associated current-correction value *I₁, I₂*=0 or *I₃*, and transmits the latter to a summation module 602. The latter receives the load signals emitted by the voltage detector 163, which signals represent the current *I*ₘₒₜ through the motor 122. In the summation module 602, the selected current-correction value *Iᵢ (i* = 1, 2, 3) is subtracted from these load signals in order to compensate for acceleration influences on the load signals. The difference *I*ₘₒₜ - *Iᵢ* constitutes a good measure for the torque generated by the motor. The selection module 601 and the summation module 602 together form a correction module.

The difference established by this correction module is now averaged in a two-stage method. For this purpose, a first averaging 〈*I*ₘₒₜ *- Iᵢ*〉*_{N}* is carried out in a first submodule 603 over a fixed number of encoder counts *N*_{enc}, that is to say over a fixed (not necessarily integer) number of motor revolutions, e.g. over that number of revolutions that corresponds to a release amount of 0.1 IU. A second submodule 604 again averages these averages continuously by a moving average filter (running average). The two submodules 603, 604 together form an averaging module. The moving average filter supplies an average over a number *N*_{enc} of encoder counts, i.e. per 0.1 IU in the present example. A predefined number of these averages is continuously stored in a results table, e.g. the last 50 averages in each case. The most recent average is firstly compared in a first comparison module 607 to a predefined maximum absolute value avgₘₐₓ for the motor current. If the average exceeds the maximum absolute value, the first comparison module 607 emits an occlusion signal 608, since this indicates an abnormal load on the motor. Secondly, a difference between the most recent average and the oldest average in the table is formed in a differencing module 605, that is to say the increase of the corrected and averaged motor current is established over a particular window. Thus, in the present example (respectively one average per 0.1 IU, 50 stored averages in the table), the increase of the motor current is established over an administered amount of 50 × 0.1 IU = 5 IU. If this difference exceeds a certain maximum difference value diffₘₐₓ, a second comparison module 606 likewise emits an occlusion signal 608, since this indicates a large increase in the torque generated by the motor and hence indicates a pressure increase in the container 210 or another type of blockage. Provision can be made for this occlusion signal to be emitted only if additionally the condition is satisfied that the most recent average exceeds a certain minimum absolute value in order to avoid false alarms.

Additionally, there can be further modules (not illustrated) for further monitoring functions, e.g. a measurement module for evaluating the reaction torque established by the force sensor 170. This can likewise be carried out in the manner that an occlusion signal is emitted firstly when the reaction torque exceeds a maximum absolute value and secondly when torques at different times exceed a maximum difference value.

Figure 7 illustrates the profile of the motor speed and the motor current before being corrected and averaged, as measured for an administering apparatus of the type illustrated in Figures 1 and 2. As will be immediately apparent, the measured motor speed v (in arbitrary units a.u.) follows the predefined speed profile very closely. It can clearly be seen that the motor current, on average, is higher during the acceleration phase P₁ and by contrast, on average, is lower during the deceleration phase P₃ than during the stationary phase P₂. The current averages in the three phases of the speed profile are illustrated by horizontal lines in Figure 7. The differences of the current averages in the phases P₁ and P₃ with respect to the current average in the stationary phase P₂ constitute the correction values I₁ and I₃ used in the aforementioned method, provided the assumption is made that I₂ = 0. In particular, the correction values I₁, I₂ and I₃ are constant (possibly zero) if the speed changes in the associated phases are linear. This enables a simple correction of the dynamic error resulting from inertia.

The control of the administering apparatus as illustrated here allows a fast and reliable recognition of malfunctions that lead to a torque increase in the motor, more particularly occlusions or other blockages that can lead to an interruption of the administration. Additionally, provision can optionally be made for further measures for occlusion recognition that are well-known.

A multiplicity of modifications are possible without departing from the scope of the invention. In particular, the illustrated control is not limited to the specific administering apparatus in Figures 1 and 2. The control need not be implemented digitally, but it can also be realized completely or in part by analogue circuits. In the case of a digital implementation, the method for controlling can be implemented as software, i.e. in a computer program product, which contains computer code carrying out the steps of the method when executed on a processor. The control of the motor speed by means of a speed profile with at least one acceleration region and one deceleration region can advantageously also be used independently of the aforementioned monitoring arrangement for occlusion recognition. The occlusion recognition can even be used when the motor speed is controlled as per a speed profile, but not regulated actively via a feedback control loop. More particularly, it is also possible to define the motor speed in the profile as a function of time instead of a position difference. A multiplicity of further modifications are possible.

### LIST OF REFERENCE SIGNS

| | | | |
|---|---|---|---|
| 100 | Base unit | 161 | Motor driver unit |
| 110 | Housing | 162 | Shunt resistor |
| 111 | Operating element | 163 | Voltage detector |
| 112 | Finger-like structure | 170 | Force sensor |
| 113 | Switch | 200 | Cartridge |
| 114 | Latch | 210 | Housing |
| 120 | Battery | 220 | Product container |
| 121 | Control device | 221 | Product piston |
| 122 | Drive motor | 222 | End cap |
| 123 | Transmission | 223 | Displacement reservoir |
| 124 | Driver | 230 | Hydraulics reservoir |
| 125 | Encoder | 231 | Hydraulics piston |
| 126 | Suspension | 232 | Support |
| 130 | Main processor | 233 | Seal |
| 131 | State machine | 234 | Transmission sleeve |
| 132 | Interface | 236 | Longitudinal groove |
| 133 | Event logbook | 240 | Closure element |
| 134 | Alarm module | 241 | Fluid channel |
| 135 | Monitoring arrangement | 242 | Sealing ring |
| 140 | Supervisor processor | 250 | Guide nut |
| 141 | State machine | 601 | Selection module |
| 142 | Interface | 602 | Summation module |
| 145 | Monitoring arrangement | 603 | First submodule |
| 146 | Motor control arrangement | 604 | Second submodule |
| 147 | Memory | 605 | Differencing module |
| 150 | Display | 606 | Second comparison module |
| 151 | Vibrator | 607 | First comparison module |
| 152 | Buzzer | 608 | Occlusion signal |

## Claims

1. A device for controlling a medical administering apparatus, comprising:
a motor control arrangement (146) designed to activate an electrical motor (122) of the medical administering apparatus during defined discharging events, the motor forming an electrical load;
a load sensor (162, 163) for determining load signals that constitute a measure for the electrical load formed by the motor (122);
a monitoring arrangement (145) designed to compare a variable derived from the load signals by low pass filtering and/or averaging the load signals to at least one predefined condition, and to emit an occlusion signal if the condition is satisfied; wherein
the motor control arrangement (146) is designed to control the motor (122) with a motor speed (v) as per a predefined speed profile with a plurality of regions (P₁, P₂, P₃); and
wherein the speed profile has at least the following regions:
an acceleration region (P₁) in which the motor speed (v) substantially increases; and
a deceleration region (P₃) in which the motor speed (v) substantially decreases,
**characterized**
**in that** the monitoring arrangement (145) comprises a correction module (601, 602) for correcting, by means of one associated correction value (I₁; I₂; I₃) for each region, the load signals as a function of the region (P₁; P₂; P₃) of the speed profile in which the motor control arrangement (146) is situated.

2. The device according to Claim 1,
wherein the device has a memory (147) for storing the speed profile and in each case the one associated correction value (I₁; I₂; I₃) for each region of the speed profile,
wherein the motor control arrangement (146) is designed to read out the speed profile from the memory (147) and actuate the motor (122) as per the speed profile,
and wherein the correction module (601, 602) is designed to read out the correction values (I₁; I₂, I₃) from the memory (147) and to correct the load signals accordingly.

3. The device according to one of the preceding claims, comprising a position sensor (125) for establishing an actual position of the motor (122) or of a drive component (123, 124) connected thereto.

4. The device according to Claim 3, wherein the speed profile is at least in part defined by virtue of the fact that intended speed values (v) are predefined as a function of a position deviation (Δx) of the actual position from an intended position.

5. The device according to one of the preceding claims, which comprises an arrangement for determining the motor speed (v), the control arrangement (146) comprising a speed controller, which receives the determined motor speed (v) as a control variable and intended speed values as per the speed profile as a reference variable and outputs a manipulated variable for the motor (122) for regulating the motor speed (v) as per the speed profile.

6. The device according to one of the preceding claims, wherein the monitoring arrangement (145) comprises an averaging module for forming averages or sums from the load signals.

7. The device according to Claim 6, wherein the averaging module comprises a first submodule (603) for respectively forming a first average for successive discrete movement regions of the motor, and wherein the averaging module comprises a second submodule (604) for forming second averages from the first averages by means of a running average.

8. The device according to one of the preceding claims, wherein the load sensor (162, 163) comprises a current sensor for establishing load signals representing a current flowing through the motor (122).

9. The device according to Claim 8, wherein the current sensor comprises a resistor (162) connected in series with the motor (122) and an amplifier for a voltage drop across the resistor or a Hall sensor for measuring a magnetic field generated by the current.

10. The device according to one of the preceding claims,
wherein the monitoring arrangement comprises a first comparison module (607) for emitting an occlusion signal when a variable derived from the load signals exceeds a predefined absolute value (avgₘₐₓ),
wherein the monitoring arrangement comprises a differencing module (605) for forming differences between respectively two variables derived from the load signals at different times, and
wherein the monitoring arrangement comprises a second comparison module (606) for emitting an occlusion signal when the difference exceeds a predefined difference value (diffₘₐₓ).

11. The device according to one of the preceding claims, which additionally has an alarm arrangement (150, 151, 152) for emitting one or more of the following signals as a function of the occlusion signals:
an optical alarm signal;
an acoustic alarm signal; or
a tactile alarm signal.

12. The device according to one of the preceding claims, comprising a second independent monitoring arrangement (135) designed to compare a variable derived from the load signals with at least one predefined condition and to emit an occlusion signal if the condition is satisfied.

13. A medical administering apparatus for administering a fluid medicament from a reservoir (220), comprising:
a device as per one of the preceding claims;
an electrical motor (122) interacting therewith for generating a drive movement; and
one or more drive components (123, 124), coupled to the motor (122), for transmitting the drive movement onto the reservoir (220) in order to eject the medicament from the reservoir (220).

## Patentansprüche

1. Einrichtung zur Steuerung einer medizinischen Verabreichungsvorrichtung, welche umfasst:
eine Motorsteuerungsanordnung (146), die ausgestaltet ist, einen elektrischen Motor (122) der medizinischen Verabreichungsvorrichtung während definierter Entleerungsmaßnahmen zu aktivieren, wobei der Motor eine elektrische Last bildet;
einen Lastsensor (162, 163) zur Bestimmung von Lastsignalen, die ein Maß für die durch den Motor (122) gebildete Last darstellen,
eine Überwachungsanordnung (145), die ausgestaltet ist, eine von den Lastsignalen abgeleitete Variable durch Tiefpass-Filtern und/oder Durchschnittsbestimmen der Lastsignale mit mindestens einer vorab definierten Bedingung zu vergleichen, und ein Okklusions-Signal auszugeben, wenn der Bedingung genügt wurde; worin
die Motorsteuerungs-Anordnung (146) ausgelegt ist, den Motor (122) mit einer Motorgeschwindigkeit (v) gemäß einem vorab definierten Geschwindigkeitsprofil mit mehreren Bereichen (P₁, P₂, P₃) zu steuern; und
worin das Geschwindigkeitsprofil mindestens die folgenden Bereiche aufweist:
einen Beschleunigungsbereich (P1), bei dem die Motorgeschwindigkeit (v) im Wesentlichen ansteigt; und
einen Verzögerungsbereich (P₃), bei dem die Motorgeschwindigkeit (v) im Wesentlichen abnimmt,
**dadurch gekennzeichnet, dass**
die Überwachungsanordnung (145) ein Korrekturmodul (601, 602) umfasst, um mittels eines mit jedem Bereich assoziierten Korrekturwertes (T₁, T₂, T₃) die Lastsignale als eine Funktion des Bereichs (P₁, P₂, P₃) des Geschwindigkeitsprofils, in dem sich die Motorsteuerungsanordnung (146) befindet, zu korrigieren.

2. Einrichtung nach Anspruch 1,
worin die Einrichtung einen Speicher (147) zur Speicherung des Geschwindigkeitsprofils und in jedem Fall des einen mit jedem Bereich des Geschwindigkeitsprofils assoziierten Korrekturwert (T₁, T₂, T₃) des Geschwindigkeitsprofils aufweist,
worin die Motorsteuerungsanordnung (146) ausgestaltet ist, das Geschwindigkeitsprofil aus dem Speicher (147) auszulesen und den Motor (122) gemäß dem Geschwindigkeitsprofil anzutreiben,
und worin das Korrekturmodul (601, 602) ausgestaltet ist, die Korrekturwerte (T₁, T₂, T₃) aus dem Speicher (147) auszulesen und die Lastsignale entsprechend zu korrigieren.

3. Einrichtung nach einem der vorstehenden Ansprüche, die einen Positionssensor (125) zur Feststellung der tatsächlichen Position des Motors (122) oder einer damit verbundenen Antriebskomponente (123, 124) umfasst.

4. Einrichtung nach Anspruch 3, worin das Geschwindigkeitsprofil mindestens teilweise durch die Tatsache definiert ist, dass die beabsichtigten Geschwindigkeitswerte (v) als Funktion einer Positionsabweichung (Δx) der tatsächlichen Position von einer angestrebten Position vorab definiert sind.

5. Einrichtung nach einem der vorstehenden Ansprüche, welche eine Anordnung zur Bestimmung der Motorgeschwindigkeit (v) umfasst, worin die Steueranordnung (146) einen Geschwindigkeitskontroller umfasst, der die bestimmte Motorgeschwindigkeit (v) als eine Steuervariable und die gemäß dem Geschwindigkeitsprofil geplanten Geschwindigkeitswerte als eine Bezugsvariable erhält, und eine verstellte Variable für den Motor (122) ausgibt, um die Motorgeschwindigkeit (v) gemäß dem Geschwindigkeitsprofil zu regulieren.

6. Einrichtung nach einem der vorstehenden Ansprüche, worin die Überwachungsanordnung (145) ein Durchschnittsbestimmungs-Modul umfasst, um Durchschnitte oder Summen der Lastsignale zu bilden.

7. Einrichtung nach Anspruch 6, worin das Durchschnittsbestimmungs-Modul ein erstes Submodul (603) umfasst, um jeweils einen ersten Durchschnitt für aufeinander folgende bestimmte Bewegungsbereiche des Motors zu bilden, und worin das Durchschnitts-bestimmungs-Modul ein zweites Submodul (604) umfasst, um aus den ersten Durchschnitten mittels eines fortlaufenden Durchschnitts zweite Durchschnitte zu bilden.

8. Einrichtung nach einem der vorstehenden Ansprüche, worin der Lastsensor (162, 163) einen Strom-Sensor umfasst, um Lastsignale zu bilden, die einen durch den Motor (122) laufenden Strom darzustellen.

9. Einrichtung nach Anspruch 8, worin der Strom-Sensor einen Widerstand (162) umfasst, der mit dem Motor (122) in Reihe geschaltet ist, und einen Verstärker für einen Spannungsabfall über den Widerstand oder einen Hall-Sensor zum Bestimmen eines durch den Strom erzeugten magnetischen Feldes.

10. Einrichtung nach einem der vorstehenden Ansprüche,
worin die Überwachungsanordnung ein erstes Vergleichsmodul (607) umfasst, um ein Okklusions-Signal zu auszugeben, wenn eine von den Lastsignalen abweichende Variable einen vorab bestimmten absoluten Wert (avgₘₐₓ) übersteigt,
worin die Überwachungsanordnung ein Unterscheidungsmodul (605) umfasst, um zwischen jeweils zwei von den Lastsignalen zu unterschiedlichen Zeitpunkten abgeleiteten Variablen Unterschiede zu bilden, und
worin die Überwachungsanordnung ein zweites Vergleichsmodul (606) umfasst, um ein Okklusions-Signal auszugeben, wenn der Unterschied einen vorab bestimmten Wert (diffₘₐₓ) übersteigt.

11. Einrichtung nach einem der vorstehenden Ansprüche, die weiter eine Alarmanordnung (150, 151, 152) umfasst, um ein oder mehrere der folgenden Signale als eine Funktion des Okklusions-Signals zu emittieren:
ein optisches Alarmsignal;
ein akustisches Alarmsignal; oder
ein taktiles Alarmsignal.

12. Einrichtung nach einem der vorstehenden Ansprüche, welche eine zweite unabhängige Überwachungsanordnung (135) umfasst, die angepasst ist, eine von den Lastsignalen abgeleitete Variable mit mindestens einer vorab bestimmten Bedingung zu vergleichen, und ein Okklusions-Signal auszugeben emittieren, wenn der Bedingung genügt wird.

13. Medizinische Verabreichungsvorrichtung zur Verabreichung eines fluiden Medikaments aus einem Reservoir (220), welches umfasst:
eine Einrichtung nach einem der vorstehenden Ansprüche;
einen elektrischen Motor (122), der damit wechselwirkt, um eine Antriebsbewegung zu erzeugen; und
ein oder mehrere Antriebskomponenten (123, 124), die mit dem Motor (122) gekoppelt sind, um die Antriebsbewegung auf das Reservoir zu übertragen, um das Medikament aus dem Reservoir (220) auszutreiben.

## Revendications

1. Dispositif pour commander un appareil d'administration de médicament, comprenant :
un agencement de commande de moteur (146) conçu pour actionner un moteur électrique (122) de l'appareil d'administration de médicament pendant des événements définis de décharge, le moteur formant une charge électrique ;
un capteur de charge de moteur (162, 163) pour déterminer des signaux de charge qui constituent une mesure de la charge électrique formée par le moteur (122) ;
un agencement de surveillance (145) conçu pour comparer une variable dérivée des signaux de charge par un filtrage passe-bas et/ou une moyenne des signaux de charge dans au moins une condition prédéfinie, et pour émettre un signal d'occlusion lorsque la condition est satisfaite ; dans lequel l'agencement de commande de moteur (146) est conçu pour commander le moteur (122) avec une vitesse de moteur (v) comme définie par un profil prédéfini de vitesse présentant une pluralité de régions (P₁, P₂, P₃) ; et
dans lequel le profil de vitesse comprend au moins les régions suivantes :
une région d'accélération (P₁) dans laquelle la vitesse de moteur (v) augmente sensiblement ; et
une région de décélération (P₃) dans laquelle la vitesse de moteur (v) diminue sensiblement,
**caractérisé**
**en ce que** l'agencement de surveillance (145) comprend un module de correction (601, 602) pour corriger, au moyen d'une valeur de correction associée (I₁, I₂, I₃) pour chaque région, les signaux de charge en fonction de la région (P₁, P₂, P₃) du profil de vitesse dans laquelle l'agencement de commande de moteur (146) se situe.

2. Dispositif selon la revendication 1,
dans lequel le dispositif a une mémoire (147) pour stocker le profil de vitesse et, dans chaque cas, la valeur de correction associée (I₁; I₂, I₃) de chaque région du profil de vitesse,
dans lequel l'agencement de commande de moteur (146) est conçu pour lire le profil de vitesse dans la mémoire (147) et pour actionner le moteur (122) en fonction du profil de vitesse,
et dans lequel le module de correction (601, 602) est conçu pour lire les valeurs de correction (I₁, I₂, I₃) dans la mémoire (147) et pour corriger les signaux de charge en conséquence.

3. Dispositif selon l'une quelconque des revendications précédentes, comprenant un capteur de position (125) pour établir une position réelle du moteur (122) ou d'un élément d'entraînement (123, 124) raccordé à celui-ci.

4. Dispositif selon la revendication 3, dans lequel le profil de vitesse est au moins en partie défini par le fait que les valeurs attendues de vitesse (v) sont prédéfinies en fonction d'une déviation de position (Δx) de la position réelle par rapport à une position attendue.

5. Dispositif selon l'une quelconque des revendications précédentes, qui comprend un agencement pour déterminer la vitesse de moteur (v), l'agencement de commande (146) comprenant un régulateur de vitesse, qui reçoit la vitesse déterminée de moteur (v) sous la forme d'une variable de commande et les valeurs attendues de vitesse fonction du profil de vitesse sous la forme d'une variable de référence et sort une variable réglante pour le moteur (122) afin de réguler la vitesse de moteur (v) en fonction du profil de vitesse.

6. Dispositif selon l'une quelconque des revendications précédentes, dans lequel l'agencement de surveillance (145) comprend un module de moyenne pour former des moyennes ou des sommes à partir des signaux de charge.

7. Dispositif selon la revendication 6, dans lequel le module de moyenne comprend un premier sous-module (603) pour former respectivement une première moyenne pour des régions de mouvement discrètes successives du moteur, et dans lequel le module de moyenne comprend un deuxième sous-module (604) pour former des deuxièmes moyennes à partir des premières moyennes au moyen d'une moyenne glissante.

8. Dispositif selon l'une quelconque des revendications précédentes, dans lequel le capteur de charge de moteur (162, 163) comprend un détecteur de courant pour établir des signaux de charge représentant un courant passant par le moteur (122).

9. Dispositif selon la revendication 8, dans lequel le détecteur de courant comprend une résistance (162) raccordée en série au moteur (122) et un amplificateur pour une chute de tension aux bornes de la résistance ou un capteur à effet Hall pour mesurer un champ magnétique généré par le courant.

10. Dispositif selon l'une quelconque des revendications précédentes,
dans lequel l'agencement de surveillance comprend un premier module de comparaison (607) pour émettre un signal d'occlusion quand une variable dérivée des signaux de charge dépasse une valeur absolue prédéfinie (avgₘₐₓ) ;
dans lequel l'agencement de surveillance comprend un module de différenciation (605) pour former des différences entre respectivement deux variables dérivées des signaux de charge à des moments différents, et
dans lequel l'agencement de surveillance comprend un deuxième module de comparaison (606) pour émettre un signal d'occlusion quand la différence dépasse une valeur prédéfinie de différence (diffₘₐₓ).

11. Dispositif selon l'une quelconque des revendications précédentes, qui comprend en outre un agencement d'alarme (150, 151, 152) pour émettre un ou plusieurs des signaux suivants en fonction des signaux d'occlusion :
un signal d'alarme optique ;
un signal d'alarme acoustique ; ou
un signal d'alarme tactile.

12. Dispositif selon l'une quelconque des revendications précédentes, comprenant un deuxième agencement de surveillance indépendant (135) conçu pour comparer une variable dérivée des signaux de charge dans au moins une condition prédéfinie et pour émettre un signal d'occlusion lorsque la condition est satisfaite.

13. Appareil d'administration de médicament pour administrer un médicament fluide à partir d'un réservoir (220), comprenant :
un dispositif comme celui des revendications précédentes ;
un moteur électrique (122) en interaction avec l'appareil pour générer un mouvement d'entraînement ; et
un ou plusieurs composants d'entraînement (123, 124), couplés au moteur (122), pour transmettre le mouvement d'entraînement au réservoir (220) afin d'éjecter le médicament du réservoir (220).
